# EUROPEAN PATENT SPECIFICATION

(11) **EP 4 476 195 B1**
(45) Date of publication and mention of the grant of the patent: **08.10.2025**
(21) Application number: 23702626.5
(22) Date of filing: 06.02.2023
(51) Int. Cl.: C07C 29/10, C07C 31/20, C07D 301/12

(54) **IMPROVED PROCESS FOR PRODUCING 1,2-ALKANEDIOL FROM THE CORRESPONDING ALKENE AND HYDROGEN PEROXIDE**
VERBESSERTES VERFAHREN ZUR HERSTELLUNG VON 1,2-ALKANDIOL AUS DEM ENTSPRECHENDEN ALKEN UND WASSERSTOFFPEROXID
PROCÉDÉ AMÉLIORÉ DE PRODUCTION D'UN 1,2-ALCANEDIOL À PARTIR DE L'ALCÈNE CORRESPONDANT ET DE PEROXYDE D'HYDROGÈNE

(30) Priority: 11.02.2022 EP 22156435
(43) Date of publication of application: 18.12.2024
(73) Proprietor: Evonik Operations GmbH, 45128 Essen (DE); Dow Global Technologies LLC, Midland, MI 48674 (US)
(72) Inventor: MOLITOR, Erich J., Midland, Michigan 48640 (US)
(74) Representative: f & e patent
(86) International application number: PCT/EP2023/052811
(87) International publication number: WO 2023/152083

(56) References cited:
- US-B1- 6 528 665
- US-B2- 10 214 471

## Description

The present invention relates to a process for preparing an 1,2-alkanediol from the corresponding alkene and hydrogen peroxide which does not require isolation and purification of the intermediate alkene oxide.

On an industrial scale, 1,2-alkanediols, such as propylene glycol, are traditionally prepared by first oxidizing an alkene to the corresponding alkene oxide, which is then isolated and purified before being converted to the corresponding 1,2-alkanediol by hydrolysis.

US 10,214,471 B2 relates to a process for directly producing 1,2-propanediol from propene and hydrogen peroxide using a combination of a phase transfer catalyst and a heteropolytungstate in a biphasic reaction mixture which process does not require isolation and purification of the intermediate propene oxide, leading to a reduction in equipment costs. In a first step of the described continuous process, the alkene is reacted with hydrogen peroxide in the presence of the catalyst mixture comprising a phase transfer catalyst and a heteropolytungstate in a liquid biphasic system comprising an aqueous phase having a pH of at most 6 and an organic phase. In a second step, the organic phase comprising the propene oxide is separated from the aqueous phase and recycled into the reaction mixture of the first step, and 1,2-propanediol is separated from the aqueous phase obtained in the second step. Various phase transfer catalysts, including tertiary amines and tetraalkylammonium salts, such as trimethylamine and dodecyltrimethylammonium salts, are described in US 10,214,471 B2. For instance, example 9 of US 10,214,471 describes a continuous process of preparing 1,2-propanediol from propene and hydrogen peroxide using trioctylamine with a yield of 52% in the aqueous phase.

J. Kaur *et al.* describe the epoxidation of propene with hydrogen peroxide in a microemulsion or a biphasic solvent mixture catalyzed by peroxo polytungstophosphates, which are prepared by interaction of H₂WO₄, H₂O₂ and H₃PO₄ in the presence of methyltrioctylammonium chloride as a surfactant (Catal. Commun. 2004, 5, 709-713 DOI: 10.1016/j.catcom.2004.09.004).

C. Venturello *et al.* describe a process for the epoxidation of olefins, such as 1-octene and cyclohexene, using hydrogen peroxide in the presence of a polytungstophosphate catalyst and a tetraalkylammonium phase transfer catalyst, such as methyltrioctylammonium chloride, in a biphasic solvent mixture (J. Org. Chem. 1983, 48, 3831-3833).

The formation of a stable emulsions is sometimes observed in the above biphasic processes, making the step of separating the aqueous and organic phases time- and energy-consuming.

Accordingly, it is an object of the present invention to provide an improved biphasic process for the production of an 1,2-alkanediol, such as 1,2-propanediol, from the corresponding alkene, which avoids the drawbacks of the prior art processes.

It has now surprisingly been found that the use of specific tetraalkylammonium cations in the biphasic reaction mixture avoids the formation of a stable emulsion without compromising efficiency, yield or selectivity of the epoxidation process. Said tetraalkylammonium cations have the general formula N⁺R¹R²R³R⁴, wherein R¹, R², R³, and R⁴ are alkyl groups which each independently may be the same or different, wherein, when all of R¹, R², R³, and R⁴ are the same, the tetraalkylammonium cation has in total at least 32 carbon atoms in the alkyl groups, or, when at least one of R¹, R², R³, and R⁴ is different from the others, the tetraalkylammonium cation has in total at least 37 carbon atoms in the alkyl groups.

Accordingly, the present invention relates to a process for preparing an 1,2-alkanediol from the corresponding alkene and hydrogen peroxide, the process comprising the steps of:
a) reacting the alkene with hydrogen peroxide in the presence of a catalytic amount of at least one tungsten polyoxometalate and at least one tetraalkylammonium cation,
   wherein the reaction is carried out in a biphasic reaction mixture comprising an aqueous phase having a pH of at most 6 and an organic phase,
   wherein the tetraalkylammonium cation has the general formula N⁺R¹R²R³R⁴, wherein R¹, R², R³, and R⁴ are alkyl groups which each independently may be the same or different, wherein, when all of R¹, R², R³, and R⁴ are the same, the tetraalkylammonium cation has in total at least 32 carbon atoms in the alkyl groups, or, when at least one of R¹, R², R³, and R⁴ is different from the others, the tetraalkylammonium cation has in total at least 37 carbon atoms in the alkyl groups;
b) separating the biphasic mixture from step a) into an aqueous phase P1 and an organic phase P2;
c) optionally recycling alkene oxide which may be present in the separated organic phase P2 into reaction step a); and
d) separating 1,2-alkanediol from the aqueous phase P1 separated in step b).

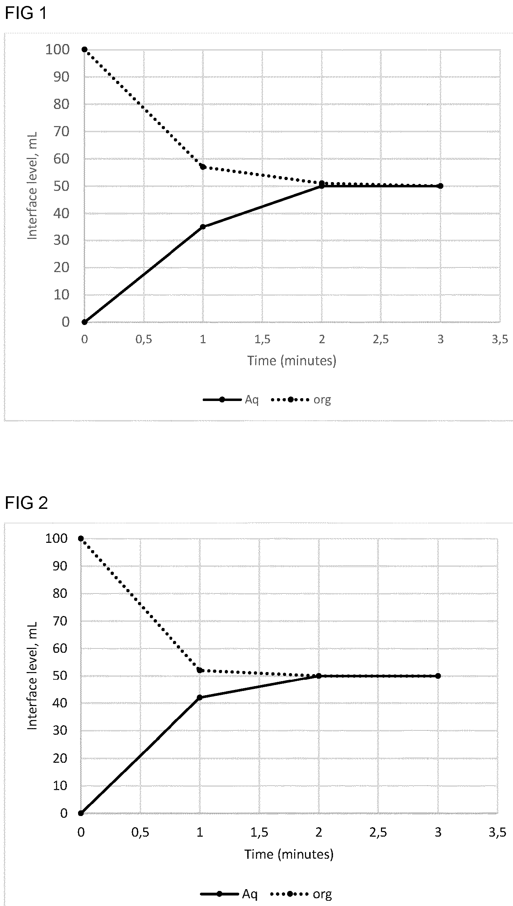

FIG. 1 shows the phase separation of a biphasic mixture according to the present invention (Example 1), using tetraoctylammonium as the tetraalkyl cation and mesitylene as the organic solvent at ambient temperature, recording the interface level (mL) of the organic phase (upper curve) and the aqueous phase (lower curve) over time (minutes).
FIG. 2 shows the phase separation of a biphasic mixture according to the present invention (Example 2), using tetraoctylammonium as the tetraalkyl cation and Hydrosol A200 ND as the organic solvent at ambient temperature, recording the interface level (mL) of the organic phase (upper curve) and the aqueous phase (lower curve) over time (minutes).

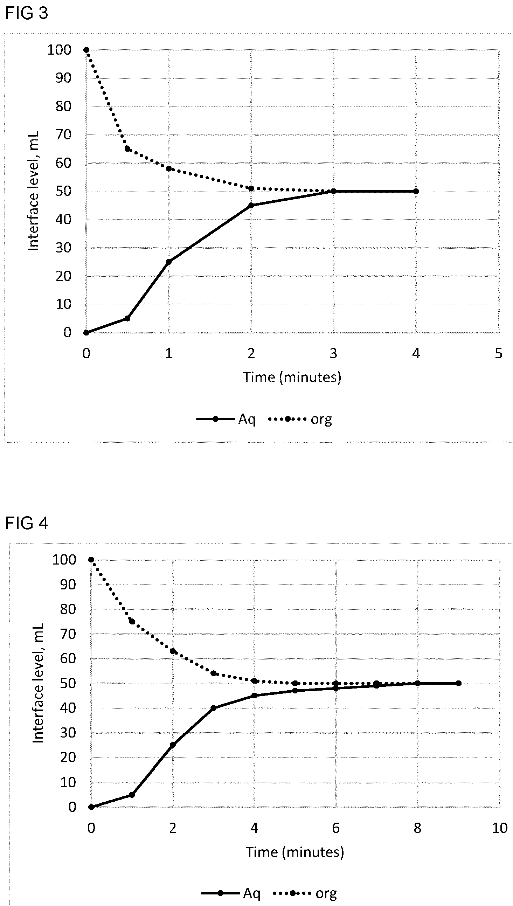

FIG. 3 shows the phase separation of a biphasic mixture according to the present invention (Example 3), using tetradecylammonium as the tetraalkyl cation and mesitylene as the organic solvent at ambient temperature, recording the interface level (mL) of the organic phase (upper curve) and the aqueous phase (lower curve) over time (minutes).
FIG. 4 shows the phase separation of a biphasic mixture according to the present invention (Example 4), using tridodecylmethylammonium as the tetraalkyl cation and mesitylene as the organic solvent at ambient temperature, recording the interface level (mL) of the organic phase (upper curve) and the aqueous phase (lower curve) over time (minutes).

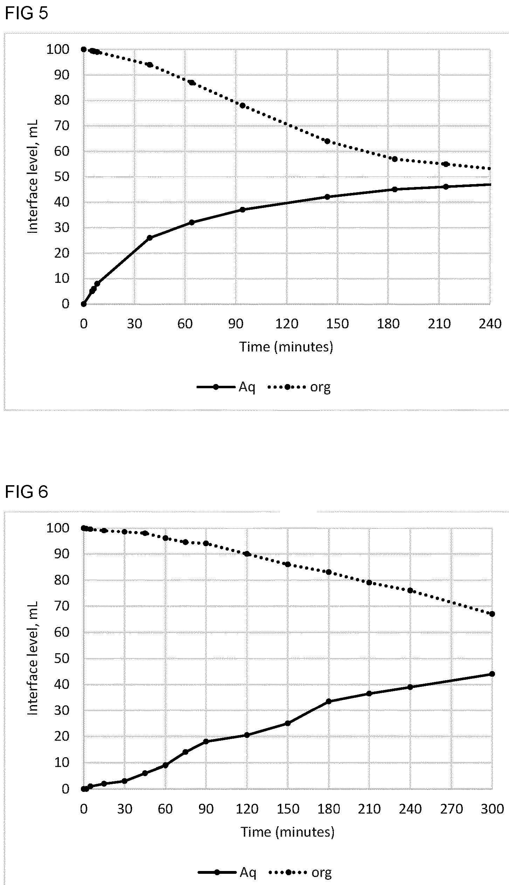

FIG. 5 shows the phase separation of a comparative biphasic mixture (Comparative Example 5), using methyltrialkyl(C8-C10) ammonium as the tetraalkyl cation and mesitylene as the organic solvent at ambient temperature, recording the interface level (mL) of the organic phase (upper curve) and the aqueous phase (lower curve) over time (minutes).
FIG. 6 shows the phase separation of a comparative biphasic mixture (Comparative Example 6), using methyltrialkyl(C8-C10) ammonium as the tetraalkyl cation and mesitylene as the organic solvent at 50°C, recording the interface level (mL) of the organic phase (upper curve) and the aqueous phase (lower curve) over time (minutes).

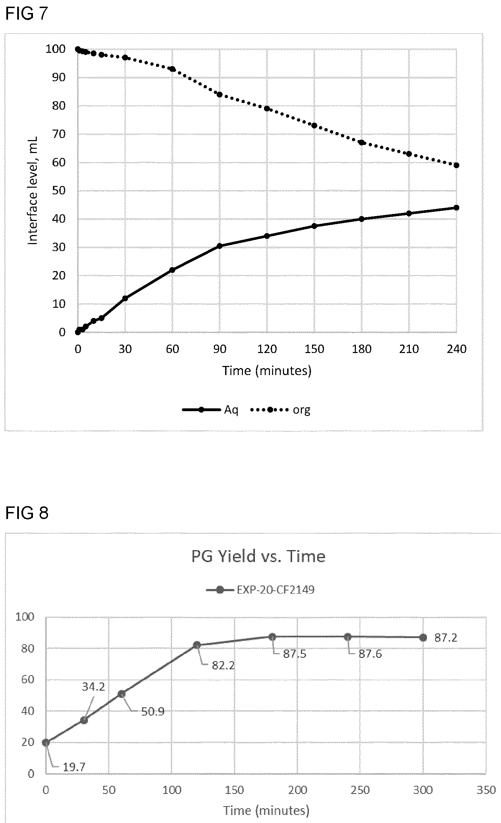

FIG. 7 shows the phase separation of a comparative biphasic mixture (Comparative Example 7), using methyltrialkyl(C8-C10) ammonium as the tetraalkyl cation and Hydrosol A200 ND as the organic solvent at ambient temperature, recording the interface level (mL) of the organic phase (upper curve) and the aqueous phase (lower curve) over time (minutes).
FIG. 8 shows the mol% yield of 1,2-propanediol obtained in an epoxidation / hydrolysis process according to the present invention (Example 8) as a function of reaction time (minutes).

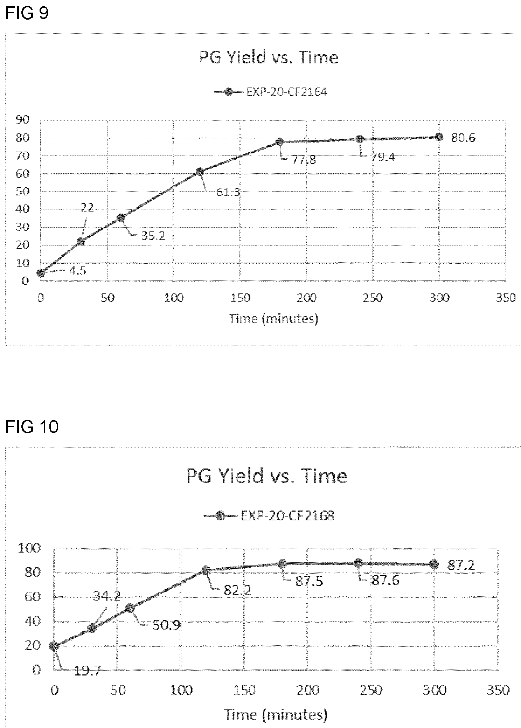

FIG. 9 shows the mol% yield of 1,2-propanediol obtained in an epoxidation / hydrolysis process according to the present invention (Example 9) as a function of reaction time (minutes).
FIG. 10 shows the mol% yield of 1,2-propanediol obtained in an epoxidation / hydrolysis process according to the present invention (Example 10) as a function of reaction time (minutes).

As used herein, the term "comprising" and variations thereof are used synonymously with the terms "including", "containing" and variations thereof and are understood to be open and non-limiting terms which do not exclude the presence of additional undescribed or unrecited elements, compounds, ingredients or process steps. As used herein, the term "consisting of" is understood to exclude the presence of unspecified elements, compounds, ingredients or process steps. When the non-limiting terms "comprising", "including" or "containing" are used in the present specification, the case of "consisting of" is included therein. For example, a process described as "comprising" or "including" certain steps can consist of the explicitly recited steps or can further comprise one or more unrecited steps. Same applies, for example, to compositions and their corresponding explicitly described or non-recited ingredients.

As used herein, the term "alkene" is understood to be an unsaturated (having a carbon-carbon double bond) acyclic branched or unbranched hydrocarbon of the general formula CₙH₂ₙ or a cyclic hydrocarbon of the general formula CₙH₂ₙ₋₂. Suitable alkenes include, but are not limited to, propene, butene, hexene, octene, and cyclohexene. The alkene may preferably be propene.

As used herein, the term "alkyl" is understood to be an acyclic, saturated, branched or unbranched, preferably unbranched, group consisting of hydrogen and carbon having the general formula CₙH₂ₙ₊₁. The alkyl group might be substituted or unsubstituted, but preferably is unsubstituted.

Unless stated otherwise, ambient temperature refers to room temperature (23 °C). In step a) of the process of the present invention the alkene is reacted with hydrogen peroxide in the presence of a catalytic amount of at least one tungsten polyoxometalate and at least one tetraalkylammonium cation. The reaction is carried out in a liquid mixture comprising two liquid phases, an aqueous phase having a pH of at most 6 and an organic phase.

The alkene can be used in pure form or as a mixture with the corresponding alkane, wherein the proportion of the alkane may be up to 20 mol%, but preferably is less than 5 mol%, based on the combined amount of alkene and alkane.

Hydrogen peroxide may preferably be used in the form of an aqueous solution, preferably with a hydrogen peroxide content of 10 to 80% by weight, particularly preferably 30 to 70% by weight, based on the total weight of the aqueous hydrogen peroxide solution. The hydrogen peroxide may be prepared by every conceivable method, including, but not limited to an anthraquinone process. For instance, the hydrogen peroxide crude product obtained in the extraction stage of the anthraquinone process for producing hydrogen peroxide may be used.

During the course of the reaction, the aqueous phase will comprise water, unreacted hydrogen peroxide and the 1,2-alkanediol formed during the reaction. The organic phase will comprise the alkene and alkene oxide formed as intermediate. If a mixture of an alkene and the corresponding alkane, such as propene and propane, is used, the organic phase can in addition comprise the alkane. Further, the organic phase may comprise at least one solvent immiscible with water.

The reaction mixture of the present invention comprises a tungsten polyoxometalate, preferably a heteropolytungstate. The heteroatom of the heteropolytungstate may preferably be phosphorus or arsenic and may particularly preferably be phosphorus, i.e., the heteropolytungstate may particularly preferably be a polytungstophosphate. Special preference is given to polytungstophosphates having a molar ratio of phosphorus to tungsten in the range of from 1:2 to 1:12. A preformed tungsten polyoxometalate may be added to the reaction in step a) or the tungsten polyoxometalate may be generated in *situ* in the liquid reaction mixture of step a). For instance, a polytungstophosphate may preferably be generated *in situ* from phosphoric acid and a water-soluble alkaline tungstate, preferably sodium tungstate, wherein phosphoric acid and alkaline tungstate may preferably be used at a molar ratio of phosphorus to tungsten in the range of from 1:2 to 10:1 and particularly of from 3:1 to 8:1. Peroxotungstates and peroxotungstophosphates, for example PO₄[WO(O₂)₂]₄³⁻ and HPO₄[WO(O₂)₂]₂²⁻ and also partially protonated forms thereof, are formed from a polytungstophosphate with hydrogen peroxide in the aqueous phase.

The reaction mixture used in the process according to the invention further comprises at least one tetraalkylammonium cation. The tetraalkylammonium cation has the general formula N⁺R¹R²R³R⁴, wherein R¹, R², R³, and R⁴ are alkyl groups which each independently may be the same or different, wherein, when all of R¹, R², R³, and R⁴ are the same, the tetraalkylammonium cation has in total at least 32 carbon atoms in the alkyl group, or, when at least one of R¹, R², R³ and R⁴ is different from the others, the tetraalkylammonium cation has in total at least 37 carbon atoms in the alkyl group. If a mixture of different tetraalkylammonium cations is used, the average number of total carbon atoms preferably also is at least 32 for those tetraalkylammonium cations wherein all of R¹, R², R³, and R⁴ are the same and at least 37 for those tetraalkylammonium cations wherein at least one of R¹, R², R³ and R⁴ is different from the others.

When at least one of R¹, R², R³ and R⁴ is different from the others, R¹ preferably may be CH₃ while R², R³ and R⁴ may be the same, and preferably may be the same C₁₂-C₁₈ alkyl group. When all of R¹, R², R³ and R⁴ are the same, each of R¹, R², R³ and R⁴ may preferably be the same C₈-C₁₈ alkyl. The tetraalkylammonium cation may comprise a tetraalkylammonium cation which comprises at least one of tetraoctylammonium, tetradodecylammonium, tridodecylmethylammonium, or a mixture thereof.

The tetraalkylammonium cation can form an organic phase soluble salt with the tungsten polyoxometalate.

The tetraalkylammonium cation may preferably be added to the reaction in form of a quaternary tetraalkylammonium salt having an anion different from the tungsten polyoxometalate, such as, but not limited to, a tetraalkylammonium halide (e.g., a chloride, a bromide or an iodide), sulfate or methyl sulfate. Using a salt having methyl sulfate as anion, the corrosivity of the reaction mixture can be reduced in comparison to tetraalkylammonium halides. The tungsten polyoxometalate and tetraalkylammonium salt can be fed to the reaction in step a) as a mixture or separately from each other, wherein separate feeding be preferred. However, it is also possible to add a preformed quaternary tetraalkylammonium tungsten polyoxometalate, wherein the tungsten polyoxometalate represents the counterion of the quaternary tetraalkyl ammonium cation.

The tetraalkylammonium cation may preferably be used in an amount which results in a molar ratio in the liquid mixture of tetraalkylammonium cation to tungsten in the range of from 1:1 to 3:1.

The reaction of the alkene with hydrogen peroxide is carried out at a pH of the aqueous phase of at most 6. The pH of the aqueous phase may preferably be maintained in the range from 1.0 to 3.5, particularly preferably in the range from 2.0 to 3.0, determined at room temperature. The pH can be maintained in this range by addition of acid, preferably sulphuric acid or phosphoric acid, or by addition of base, preferably aqueous sodium hydroxide solution. The term pH here refers to the apparent pH measured with a glass electrode, wherein the glass electrode has been calibrated with aqueous buffer solutions. By adjusting to a pH in the preferred range, high selectivity for 1,2-alkanediol can be achieved and enrichment of the corresponding alkene oxide in the aqueous phase can be prevented, which simplifies the subsequent separation of 1,2-alkanediol from the aqueous phase.

The reaction of the alkene with hydrogen peroxide may preferably carried out with a molar excess of the alkene, wherein the alkene may preferably be used in a molar ratio of alkene to hydrogen peroxide of from 1.1:1 to 10:1.

The reaction may preferably be carried out at a temperature in the range of 30 to 100° C, particularly preferably 70 to 90° C. The reaction may preferably carried out at a pressure which is higher than the saturated vapor pressure of the alkene at the temperature of the reaction such that the major portion of the alkene is present in the organic phase of the liquid mixture.

The reaction of alkene with hydrogen peroxide can be carried out with or without the addition of solvents. The reaction may preferably be carried out in the presence of at least one solvent having a boiling point of more than 100° C, preferably more than 120° C, and a water solubility at 20° C of less than 0.5 g/kg, preferably less than 250 mg/kg. The solvents used may be halogenated hydrocarbons, such as 1,2-dichloroethane, and/or aromatic hydrocarbons, such as alkylated aromatic hydrocarbons. It may be preferred that neither the solvent nor any other compound present in the reaction mixture of step a) comprises an ester functionality. The solvent may preferably comprise or be an aromatic hydrocarbon, such as an alkylated aromatic hydrocarbon having 7 to 12 carbon atoms or a mixture of such alkylated aromatic hydrocarbons. Suitable alkylated aromatic hydrocarbons are, for example, toluene, 1,2-dimethylbenzene (o-xylene), 1,3-dimethylbenzene (m-xylene), 1,4-dimethylbenzene (p-xylene), ethylbenzene, 1,2,3-trimethylbenzene, 1,2,4-trimethylbenzene, 1,3,5-trimethylbenzene (mesitylene), 1-ethyl-2-methylbenzene, 1-ethyl-3-methylbenzene and 1-ethyl-4-methylbenzene, n-propylbenzene, and 1,2,3,4-tetrahydronaphthalene as well as mixtures thereof. Mixtures of hydrocarbons comprising more than 50% by weight, based on the total weight of the solvent mixture, preferably more than 80% by weight, of alkylated aromatic hydrocarbons having 7 to 12 carbon atoms may preferably be used as solvent. By using a solvent comprising an alkylated aromatic hydrocarbon having 7 to 12 carbon atoms, an extensive extraction of the heteropolytungstate in form of a tetraalkylammonium complex into the organic phase of the reaction mixture can be achieved so that an improved recycling of the heteropolytungstate with the organic phase and a simplified recovery of heteropolytungstate from the aqueous phase of the reaction of alkene with hydrogen peroxide can be achieved.

The proportion of solvent may preferably be selected such that the proportion of solvent in the organic phase during the reaction is in the range of from 10 to 90% by weight, based on the total weight of the organic phase.

The reaction of the alkene with hydrogen peroxide may be carried out batchwise or continuously, wherein a continuous process may be preferred. In a continuous process, the concentration of hydrogen peroxide in the aqueous phase preferably may be in the range of from 0.1 to 5% by weight, particularly preferably of from 0.5 to 3% by weight, based on the total weight of the aqueous phase. Such a concentration of hydrogen peroxide may be adjusted by the selection of the reaction temperature, the molar ratio of alkene to hydrogen peroxide and the residence time of the liquid mixture in the reactor in which the reaction takes place.

During the reaction, the liquid mixture is preferably mixed in order to generate a large phase interface between the aqueous phase and the organic phase. For this purpose, the reaction is preferably carried out continuously in a loop reactor having fixed internals, and the liquid mixture is conducted through the loop reactor at a flow rate which generates a turbulent flow over the internals. A loop reactor suitable for carrying out the process of the present invention continuously is described in detail in US patent 10,214,471 B2, in particular in Figures 1 to 4 and column 9, lines 42 to column 10, line 51 thereof.

In step b) of the process of the present invention, the biphasic reaction mixture from step a) is separated into an aqueous phase P1 and an organic phase P2. The separation may preferably carried out in a settler vessel, and the biphasic mixture can be passed over a coalescer element comprising a structured packing or random packing with a surface wetted by the phase present dispersed in the biphasic mixture, in order to assist the separation.

The liquid phase may preferably be separated in step b) in the presence of a gas phase. The reaction in step a) can lead to a decomposition of hydrogen peroxide with formation of oxygen and in step b) the gas phase may then comprise oxygen. In order to avoid the formation of a flammable gas phase, the oxygen content of this gas phase is therefore preferably maintained below 7% by volume in step b), preferably by supplying inert gas and withdrawing a gas stream. The inert gas used may be nitrogen, argon, carbon dioxide or methane, wherein nitrogen is preferred.

If present, in optional step c) of the process of the present invention, the alkene oxide present in the organic phase P2 is recycled into the reaction of step a), in order to achieve as far as possible complete conversion of the alkene to 1,2-alkanediol. If the process of the present invention is run continuously, step c) preferably is present. Preferably, the tungsten polyoxometalate present in the organic phase P2 may additionally be recycled into the reaction of step a). Likewise, the alkene present in the organic phase P2 may preferably be recycled into the reaction of step a). If the alkene is used as a mixture with the corresponding alkane, the same amount of the alkane is preferably separated from the organic phase P2 in the recycling into step a), which is fed to step a) with the mixture of the alkene with the alkane. This way, enrichment of the alkane in the organic phase in step a) can be avoided when the reaction in step a) is performed continuously.

In a preferred embodiment of the process of the present invention, the organic phase P2 separated in step b) may be completely or partly recycled into the reaction of step a).

The organic phase P2 may preferably be separated completely or partly by nanofiltration in step c) into a retentate enriched in tungsten polyoxometalate and a permeate depleted in tungsten polyoxometalate and the retentate is recycled into the reaction of step a). Preferably, the entire organic phase P2 may be separated by nanofiltration into a retentate and a permeate. For the nanofiltration in step c), a nanofiltration membrane may be used which retains the tungsten polyoxometalate and the tetraalkylammonium cation in the retentate and allows the alkene to pass through with the permeate. The nanofiltration may then preferably be operated such that the concentration of tungsten polyoxometalate and the tetraalkylammonium cation in the retentate do not increase above the saturation concentration. As used herein, the term "nanofiltration" refers to a pressure-driven separation at a membrane, in which the membrane retains particles and dissolved molecules having a diameter of less than 2 nm. Membranes based on the polymers polyimide, polyether sulphone, polyamide and polydimethylsiloxane can be used for nanofiltration. Suitable nanofiltration membranes are commercially available, for example, from Evonik Membrane Extraction Technology MET under the name PuraMem^{®} S600, from GMT Membrantechnik under the name ONF-2, from SolSep under the designations 010306, 030306, 030705 and 030306F, and from AMS Technologies under the name NanoPro^{™} SX. Preference is given to using one of the composite membranes known from DE 195 07 584, EP 1 741 481 and WO 2011/067054.

The nanofiltration may preferably take place as a cross-flow filtration, preferably at a temperature in the range of from 20 to 90 °C, particularly preferably of from 40 to 80 °C. The transmembrane pressure may preferably range from 2 to 5 MPa. The pressure on the retentate side can be up to 10 MPa. The pressure on the permeate side may preferably be selected to be higher than the lowest pressure in steps a) and b) of the process in order to prevent outgassing of dissolved components on the permeate side.

The organic phase P2 may also be separated by nanofiltration into a retentate enriched in tungsten polyoxometalate and a permeate depleted in tungsten polyoxometalate, a stream S1 comprising unreacted alkene and alkene oxide formed as intermediate is separated by distillation from the permeate and this stream may be recycled into the reaction of step a). The distillation may preferably be carried out at a pressure at which the alkene can be condensed with the distillate by cooling with water. Alternatively, the distillation may also be carried out at a lower pressure, alkene oxide and only a portion of the alkene can be condensed with the distillate and the remaining vapor can be compressed to condense the alkene. In this embodiment, high-boiling by-products and degradation products of the phase transfer catalyst can be discharged with the bottom product of the distillation, and enrichment of poorly water-soluble by-products and impurities in the organic phase are avoided in step a) when the reaction in step a) is performed continuously. Removing stream S1 by distillation after the nanofiltration prevents further reaction of alkene oxide, formed as intermediate, with the catalyst system by heating, which leads to by-products.

If in step a) of the process the reaction is carried out in the presence of a solvent, it is preferred that, subsequent to the distillation for separating stream S1, the bottom product of this distillation is fed to a further distillation in which the solvent is separated by distillation. The solvent separated can be recycled into step a). If the alkene is used as a mixture with the corresponding alkane, the distillation may preferably be carried out so that, in addition to stream S1, a further stream is obtained, consisting essentially of the alkene and the alkane, from which alkene oxide is separated. The alkane may be completely or partly separated from this further stream and the resulting alkene obtained, separated from alkane or depleted in alkane, may preferably be recycled into step a). In this case, preferably the same amount of alkane is removed, which is fed to step a) with the mixture of alkene and alkane. The distillation of the permeate may be carried out in two stages for this purpose, where in the first distillation stage, the further stream consisting largely of alkene and alkane is separated, and subsequently stream S1 is separated in the second distillation stage. The distillation however is preferably carried out in only one column with a side draw, stream S1 being removed as a side stream and the further stream consisting essentially of alkene and alkane being removed as overhead product of the column.

The organic phase P2 may also be separated by distillation into a stream S1, comprising unreacted alkene and alkene oxide formed as intermediate, and a stream S2, depleted in alkene and alkene oxide, the stream S2 may be separated by nanofiltration into a retentate enriched in heteropolytungstate and a permeate depleted in tungsten polyoxometalate and the stream S1 may be recycled into the reaction of step a). This embodiment is preferably used if the reaction in step a) of the process is carried out in the presence of a solvent and the distillation is then carried out such that the solvent remains in stream S2. The solvent can then be removed from the permeate of the nanofiltration, preferably by distillation, and can then be recycled into the reaction of step a).

Compared to the embodiment described above, the embodiment with a separation of stream S1 prior to the nanofiltration has the advantage that a considerably smaller stream is separated by the nanofiltration, which reduces the apparatus size and the energy consumption of the nanofiltration. If the alkene is used as a mixture with alkane, in this embodiment preferably a further stream may be separated from stream S1 by an additional distillation, which consists essentially of alkene and alkane and from which alkene oxide is separated, before stream S1 is recycled into step a). The alkane may be completely or partly separated from this further stream and the resulting alkene obtained, separated from alkane or depleted in alkane, is preferably recycled into step a). In this case, preferably the same amount of alkane is separated which is fed to step a) with the mixture of alkene and alkane. For a continuous process comprising step c) reference is made US 10,214,471 B2, in particular the Figures and passages already cited above.

In step d) of the process of the present invention, 1,2-alkanediol is separated from the aqueous phase P1 separated in step b). The 1,2-alkanediol can be separated from the aqueous phase by distillation, preferably by a two-stage distillation, in which water is distilled off in the first stage and 1,2-alkanediol is distilled off in the second stage from the bottom product of the first stage.

Prior to the separation of 1,2-alkanediol, peroxides may preferably be removed by a catalytic hydrogenation. The hydrogenation may preferably be carried out using a supported hydrogenation catalyst comprising one or more metals from the group of Ru, Rh, Pd, Pt, Ag, Ir, Fe, Cu, Ni and Co on a support, wherein activated carbon, SiO₂, TiO₂, ZrO₂, Al₂O₃ and aluminum silicates may be preferred as support. Preference is given to hydrogenation catalysts comprising ruthenium as active metal. The catalytic hydrogenation may preferably be carried out at a partial hydrogen pressure of from 5 to 300 bar and a temperature of from 80 °C to 180 °C, preferably of from 90 °C. to 150° C. The hydrogenation catalyst may be used as a suspension or as a fixed bed, a trickle bed hydrogenation with a fixed bed catalyst being preferred. The hydrogenation can prevent problems due to decomposition of hydrogen peroxide in a separation of 1,2-alkanediol by distillation and reduce the by-products 1-hydroperoxy-2-alkanol, 2-hydroperoxy-1-alkanol and 1-hydroxy-2-alkanon formed in step a) to 1,2-alkanediol, and can thereby improve the yield of 1,2-alkanediol.

In step d) of the process of the present invention, the aqueous phase P1 may preferably be separated by nanofiltration into a retentate enriched in tungsten polyoxometalate and a permeate depleted in tungsten polyoxometalate, wherein the retentate may be recycled into the reaction in step a) and the 1,2-alkanediol is separated from the permeate. For the nanofiltration in step d), a nanofiltration membrane may be used which retains tungsten polyoxometalate in the retentate and allows water and 1,2-propanediol to pass through with the permeate. The nanofiltration may be operated so that the solubility limit of the tungsten polyoxometalate in the retentate is not exceeded. For a continuous reaction in step a), preferably that much water is recycled to step a) with the retentate that a concentration of 1,2-alkanediol in the aqueous phase P1 in the range of 10 to 30% by weight results. By an appropriate recycling of water, on the one hand formation of 1,1'-oxydi-2-alkanol and trialkylene glycol in step a) can be prevented and on the other hand the amount of water, which has to be separated from 1,2-alkanediol by distillation, can be kept low.

In addition to the nanofiltration or as an alternative thereto, tungsten polyoxometalate can be removed from the aqueous phase P1 by adsorption on a support material. For such an adsorption, preference is given to using a cationized inorganic support material described in WO 2009/133053 A1 on page 7, line 1 to page 8, line 29. The adsorption on the support material and the recovery of tungsten polyoxometalate adsorbed on the support material may preferably be carried out using the methods described in WO 2009/133053 A1 and WO 2013/110419 A1. If the adsorption is used in addition to a nanofiltration in step d), the adsorption may preferably be carried out after the nanofiltration in order to keep the demand for support material low.

Nanofiltration and adsorption on a support material may preferably be carried out before the hydrogenation described above, in order to avoid deactivation of the hydrogenation catalyst by tungsten polyoxometalate.

In a preferred embodiment of the process according to the invention, the aqueous phase P1 separated in step b) may be brought into contact with liquid alkene in step d) to obtain an aqueous phase P3 and an organic phase P4, the organic phase P4 may be recycled into the reaction of step a) and the 1,2-alkanediol is separated from the aqueous phase P3. In this embodiment, preferably no separation of tungsten from the aqueous phase P1 is carried out and tungsten is separated from the aqueous phase P3, preferably by nanofiltration and/or adsorption as described above for the aqueous phase P1. The aqueous phase P1 is brought into contact with alkene preferably in an additional mixed reactor with a residence time in the reactor which leads to a conversion of the hydrogen peroxide present in the aqueous phase of at least 50%, preferably at least 80%. The bringing into contact in the additional reactor is carried out preferably at a temperature of 60 to 100° C and a pressure which is above the saturated vapor pressure of alkene at the selected temperature. The aqueous phase P1 may preferably be brought into contact with alkene in a continuously operated reactor, particularly preferably in a loop reactor. By the use of an additional continuously operated reactor, a high conversion of hydrogen peroxide can be achieved with an overall lower reactor volume.

Herein, the alkene is preferably supplied to the process only in step d) and gets to the reaction of step a) with the organic phase P4. If a tetraalkylammonium cation is used in the process which, in the presence of hydrogen peroxide, transfers more than half of the tungsten from the aqueous phase into liquid alkene, the aqueous phase P1 may also be brought into contact with liquid alkene in a countercurrent extraction, preferably a countercurrent extraction column, and tungsten present in the aqueous phase P1 is then recycled into step a) with the organic phase P4.

### EXAMPLES

### I. Phase Separation Experiments:

### Examples 1 to 4 and Comparative Examples 5 to 7:

For studying the phase separation behavior of biphasic epoxidation reaction mixtures, an aqueous solution A comprising the 1,2-alkanediol and an organic solution D comprising the epoxidation catalyst and a tetraalkylammonium salt were prepared and mixed as described below. The phase separation after mixing was observed by recording the volumes of the separated organic and aqueous layers over time. The results are shown in Table 1 and Figures 1 to 7.

### Preparation of aqueous solution A:

A solution of 1,2-propanediol (20 wt.%) and phosphoric acid (0.06 wt.%) in HPLC grade demineralized water was prepared by mixing.

### Preparation of POM catalyst solution B:

Sodium tungstate dihydrate (13.0 g, 39.4 mmol) was dissolved in HPLC grade demineralized water (62.4 g). The solution was stirred at ambient temperature with a PFTE coated magnetic stir bar while 85% phosphoric acid (20.8 g, 0.181 mol) was added gradually.

### Preparation of tetraalkylammonium solution C:

The respective tetraalkylammonium salt (see Table 1) (10 mmol) was dissolved in an organic solvent (60 g).

### Preparation of tetraalkylammonium / POM catalyst solution D:

The entire tetraalkylammonium solution C was combined with 22.2 g of the POM catalyst solution B. The resulting biphasic solution was mixed with a PFTE coated magnetic stir bar for 15 minutes at ambient temperature. The phases were allowed to separate and the organic phase was recovered and used in the phase separation experiments described in the following.

### Experimental procedure for the phase separation experiments:

Equal volumes of tetraalkylammonium / POM catalyst solution D and aqueous solution A were placed in a 100 mL graduated cylinder with a 29/42 ground glass joint on top. The contents were mixed at ambient temperature by inverting the cylinder by hand and returning it to the upright position five times.

**Table 1: Phase separation experiments**

| **Example** | **Tetraalkylammonium salt** | **Organic solvent** | **Time to achieve complete phase separation** |
|---|---|---|---|
| Ex 1 | Tetraoctylammonium bromide | Mesitylene | < 3 min |
| Ex 2 | Tetraoctylammonium bromide | Hydrosol A200 ND¹ | < 3 min |
| Ex 3 | Tetradecylammonium bromide | Mesitylene | < 4 min |
| Ex 4 | Tridodecylmethylammonium iodide | Mesitylene | < 9 min |
| CE 5 | Adogen 464² | Mesitylene | > 240 min |
| CE 6³ | Adogen 464 | Mesitylene | > 300 min |
| CE 7 | Adogen 464 | Hydrosol A200 ND | > 240 min |
| ¹: Low naphthalene C10-aromatic compound mixture (from DHC Solvent Chemie GmbH, Mülheim an der Ruhr, Germany) | | | |
| ²: Methyl trialkyl (C8-C10) ammonium chloride (from Sigma Aldrich, Inc., St. Louis, MO, United States) | | | |
| ³: In this example the two phases were collected in a bottle and warmed in an oven overnight at 50 °C. The phases were transferred to a graduated cylinder and mixed at 50 °C. The phase separation was recorded at 50 °C. | | | |

When a methyltrialkylammonium salt having a total number of carbon atoms of less than 32 in the alkyl groups is used as a phase transfer catalyst, formation of a stable emulsion is observed (Comparative Examples 5 - 7). The aqueous and organic phases separate slowly regardless of the temperature and organic solvent used. Surprisingly, formation of a stable emulsion can be avoided and the time required to achieve a complete phase separation can be significantly reduced when using a tetraalkylammonium salt having with the general formula N⁺R¹R²R³R⁴, wherein, when all of R¹, R², R³, and R⁴ are the same, the tetraalkylammonium cation has in total at least 32 carbon atoms in the alkyl groups, such as tetraoctylammonium or tetradecylammonium (Examples 1 - 3), or, when at least one of R¹, R², R³, and R⁴ is different from the others, the tetraalkylammonium cation has in total at least 37 carbon atoms in the alkyl groups, such as tridodecylmethylammonium (Example 4).

### II. Epoxidation / Hydrolysis Experiments:

### Example 8:

A 1-L jacketed glass reactor was used for the catalyst preparation procedure which contained a dual pitch blade impeller agitator, bottom drain, thermoprobe, temperature-controlled heater/chiller bath, and nitrogen purge.

### Preparation of POM Catalyst Solution E:

To a glass container was added sodium tungstate dihydrate (13.85 g) and 66.35 g HPLC grade demineralized water to form a clear solution. Next, 85% phosphoric acid (22.1 g) was added with stirring. Finally, 50 wt.% aqueous hydrogen peroxide (19.4 g) was added with stirring.

### Preparation of tetraoctylammonium solution F:

To a separate bottle was added the tetraoctylammonium bromide (25.0 g) and mesitylene (253.6 g), and the mixture was stirred with heating until the tetraoctylammonium bromide was fully dissolved. This solution was loaded to the 1-L glass reactor and held at 50-60 °C. The solution was washed with a 50 wt.% sulfuric acid solution five times and then with water to effect ion exchange of bromide to sulfate. The wash solution masses are recorded in Table 2.

**Table 2. Aqueous washing steps during Epoxidation / Hydrolysis Catalyst Preparation**

| **Aqueous Wash Solution** | **Aqueous Mass Charged (g)** | **Aqueous Mass Removed (g)** |
|---|---|---|
| 50 wt.% sulfuric acid | 258 | 235 |
| 50 wt.% sulfuric acid | 261 | 267 |
| 50 wt.% sulfuric acid | 257 | 259 |
| 50 wt.% sulfuric acid | 250 | 238 |
| 50 wt.% sulfuric acid | 253 | 259 |
| Demineralized water | 163 | 144.5 |

The ion-exchanged tetraalkylammonium solution F remained in the reactor and to it was added the POM catalyst solution E (120.5 g). The mixture was stirred for 30 minutes at 55 °C. Agitation was stopped and the phases were allowed to separate. The aqueous phase was removed (136 g) and the organic solution (278.5 g) was collected and stored in a refrigerator until further use.

### Epoxidation / Hydrolysis Reaction Procedure:

A 1-L stainless steel reactor was used for the epoxidation / hydrolysis reaction, which contained a bottom drain, internal thermoprobe, heating mantle, internal cooling coils, a pitched blade impeller, and sampling line. A D1000 Isco pump was used to load propene.

The epoxidation / hydrolysis catalyst solution (78.9 g) was diluted with mesitylene (11.1 g) and then loaded to the 1-L stainless steel reactor. Next, a 15 wt.% aqueous hydrogen peroxide solution containing phosphoric acid was charged to the reactor (this solution was prepared by mixing 93.8 g 30 wt.% hydrogen peroxide, 93.8 g demineralized water, and 0.123 g 85% phosphoric acid). The reactor was sealed and pressurized with nitrogen (100 psig (689 kPa)) and vented for 2 cycles, leaving the reactor filled with nitrogen (100 psig (689 kPa)) and the filling and venting cycles. Next, liquid propene (139 mL) was charged to the reactor volumetrically. The reactor was heated to 70 °C using the electrical heating jacket and the reaction mixture was stirred at 500 rpm. Liquid samples were collected throughout the 300-minute experiment, and the concentration of 1,2-propanediol in the aqueous phase was measured by gas chromatography. The 1,2-propanediol yield (mol%) was calculated according to the following formula: 1,2-propanediol yield (mol%) = total 1,2-propanediol (moles) / total hydrogen peroxide charged (moles) * 100 wherein total 1,2-propanediol (moles) = [1,2-propanediol concentration (wt.%) * reactor aqueous phase solution mass (g)] / molecular weight of 1,2-propanediol (g/moles)

The results are summarized in Figure 8. The first sample was collected once the reactor temperature had reached a temperature within 2 °C of the desired setpoint; this first sample was labeled as 0 minutes and all subsequent samples were labeled chronologically from this first sample. As shown in Fig. 8 the catalyst composition produces a high yield (87.2%) of 1,2-propanediol.

### Example 9:

A 1-L jacketed glass reactor was used for the catalyst preparation procedure and contains a dual pitch blade impeller agitator, bottom drain, thermoprobe, temperature-controlled heater/chiller bath, and nitrogen purge.

### Preparation of POM Catalyst Solution G:

To a glass container was added sodium tungstate dihydrate (13.8 g) and 66.4 g HPLC grade demineralized water to form a clear solution. Next, 85% phosphoric acid (22.1 g) was added with stirring. Finally, 50 wt.% aqueous hydrogen peroxide (19.4 g) was added with stirring.

### Preparation of tetraoctylammonium solution H:

To a separate bottle was added the tetraoctylammonium bromide (25.15 g) and mixed xylenes (254 g, reagent grade, Sigma Aldrich, St. Louis, MO, USA) and the mixture was stirred with heating until the tetraoctylammonium bromide fully dissolved. This solution was loaded to the 1-L glass reactor and held at 55 °C. The solution was washed with a 50 wt.% sulfuric acid solution five times and then with water to effect ion exchange of bromide to sulfate. The wash solution masses are recorded in Table 3.

**Table 3: Aqueous washing steps during Epoxidation / Hydrolysis Catalyst Preparation.**

| **Aqueous Wash Solution** | **Aqueous Mass Charged (g)** | **Aqueous Mass Removed (g)** |
|---|---|---|
| 50 wt.% sulfuric acid | 264 | 234 |
| 50 wt.% sulfuric acid | 257 | 259 |
| 50 wt.% sulfuric acid | 254 | 260 |
| 50 wt.% sulfuric acid | 252 | 252 |
| 50 wt.% sulfuric acid | 257 | 259 |
| Demineralized water | 187 | 193 |

The ion-exchanged tetraalkylammonium solution G remained in the reactor, and the POM catalyst solution H (121.0 g) was added. The mixture was stirred for 30 minutes at 55 °C. Agitation was stopped, and the phases were allowed to separate. The aqueous phase was removed (112 g) and the organic solution (282 g) was collected and stored in a refrigerator until further use.

### Epoxidation / Hydrolysis Reaction Procedure:

A 1-L stainless steel reactor was used for the epoxidation / hydrolysis reactor and contained a bottom drain, internal thermoprobe, heating mantle, internal cooling coils, a pitched blade impeller, and sampling line. A D1000 Isco pump was used to load propene.

The epoxidation / hydrolysis catalyst solution (80.0 g) was diluted with mixed xylenes (10.1 g) and then loaded to the 1-L stainless steel reactor. Next, a 15 wt.% aqueous hydrogen peroxide solution containing phosphoric acid was charged to the reactor (this solution was prepared by mixing 93.8 g 30 wt.% hydrogen peroxide, 93.8 g demineralized water, and 0.110 g 85% phosphoric acid). The reactor was sealed and pressurized with nitrogen (100 psig (689 kPa)) and vented for 2 cycles, leaving the reactor filled with nitrogen (100 psig (689 kPa)) and the filling and venting cycles. Next, liquid propene (139 mL) was charged to the reactor volumetrically. The reactor was heated to 70 °C using the electrical heating jacket and the reaction mixture was stirred at 500 rpm. Liquid samples were collected throughout the 300-minute experiment and the concentration of 1,2-propanediol in the aqueous phase was measured by gas chromatography. The yield of 1,2-propanediol was calculated as described above.

The results are summarized in Figure 9. The first sample was collected once the reactor temperature had reached a temperature within 2 °C of the desired setpoint; this first sample was labeled as 0 minutes and all subsequent samples were labeled chronologically from this first sample. As shown in Fig. 9 the catalyst composition produced a high yield (80.6%) of 1,2-propanediol.

### Example 10:

A 1-L jacketed glass reactor was used for the catalyst preparation procedure, which contained a dual pitch blade impeller agitator, bottom drain, thermoprobe, temperature-controlled heater/chiller bath, and nitrogen purge.

### Preparation of POM Catalyst Solution I:

To a glass container was added sodium tungstate dihydrate (13.8 g) and 66.5 g HPLC grade demineralized water to form a clear solution. Next, 85% phosphoric acid (22.1 g) was added with stirring. Finally, 50 wt.% aqueous hydrogen peroxide (19.4 g) was added with stirring.

### Preparation of tetraoctylammonium solution H:

To a separate bottle was added the tetraoctylammonium bromide (25.22 g) and toluene (254 g, HPLC grade - Fisher Scientific), and the mixture was stirred at 50 °C until the tetraoctylammonium bromide was fully dissolved. This solution was loaded to the 1-L glass reactor and held at 50 °C. The solution was washed with a 50 wt.% sulfuric acid solution seven times and then with water two times to effect ion exchange of bromide to sulfate. The wash solution masses were recorded in Table 4.

**Table 4: Aqueous washing steps during Epoxidation / Hydrolysis Catalyst Preparation.**

| **Aqueous Wash Solution** | **Aqueous Mass Charged (g)** | **Aqueous Mass Removed (g)** |
|---|---|---|
| 50 wt.% sulfuric acid | 212 | 188 |
| 50 wt.% sulfuric acid | 228 | 223 |
| 50 wt.% sulfuric acid | 215 | 217 |
| 50 wt.% sulfuric acid | 199 | 209 |
| 50 wt.% sulfuric acid | 198 | 198 |
| 50 wt.% sulfuric acid | 211 | 208 |
| 50 wt.% sulfuric acid | 211 | 211 |
| Demineralized water | 191 | 198 |
| Demineralized water | 199 | 194 |

The ion-exchanged tetraalkylammonium solution I remained in the reactor, and the POM catalyst solution J (121.5 g) was added. The mixture was stirred for 30 minutes at 55 °C. Agitation was stopped and the phases were allowed to separate. The aqueous phase was removed (121 g) and the organic solution (278.5 g) was collected and stored in a refrigerator until further use.

### Epoxidation / Hydrolysis Reaction Procedure:

A 1-L stainless steel reactor was used for the epoxidation / hydrolysis reactor, which contained a bottom drain, internal thermoprobe, heating mantle, internal cooling coils, a pitched blade impeller, and sampling line. A D1000 Isco pump was used to load propene.

The epoxidation / hydrolysis catalyst solution (79 g) was diluted with toluene (11.1 g) and then loaded to the 1-L stainless steel reactor. Next, a 15 wt.% aqueous hydrogen peroxide solution containing phosphoric acid was charged to the reactor (this solution was prepared by mixing 93.8 g 30 wt.% hydrogen peroxide, 93.8 g demineralized water, and 0.109 g 85% phosphoric acid). The reactor was sealed and pressurized with nitrogen (100 psig (689 kPa)) and vented for 2 cycles, leaving the reactor filled with nitrogen (100 psig (689 kPa)) and the filling and venting cycles. Next, liquid propene (139 mL) was charged to the reactor volumetrically. The reactor was heated to 70 °C using the electrical heating jacket and the reaction mixture was stirred at 500 rpm. Liquid samples were collected throughout the 300-minute experiment, the concentration of 1,2-propanediol in the aqueous phase was measured by gas chromatography and the yield of 1,2-propanediol was calculated as described above.

The results are summarized in Figure 10. The first sample was collected once the reactor temperature had reached a temperature within 2 °C of the desired setpoint; this first sample was labeled as 0 minutes and all subsequent samples were labeled chronologically from this first sample. As shown in Fig. 10 the catalyst composition produced a high yield (87.2%) of 1,2-propanediol.

As demonstrated in examples 8 to 10, using the described catalyst system, 1,2-propanediol can surprisingly be produced with a significantly improved yield of 80% and more.

## Claims

1. A process for preparing an 1,2-alkanediol from the corresponding alkene and hydrogen peroxide, comprising the steps of:
a) reacting the alkene with hydrogen peroxide in the presence of a catalytic amount of at least one tungsten polyoxometalate and at least one tetraalkylammonium cation,
wherein the reaction is carried out in a biphasic reaction mixture comprising an aqueous phase having a pH of at most 6 and an organic phase,
wherein the tetraalkylammonium cation has the general formula N⁺R¹R²R³R⁴, wherein R¹, R², R³, and R⁴ are alkyl groups which each independently may be the same or different, wherein, when all of R¹, R², R³, and R⁴ are the same, the tetraalkylammonium cation has in total at least 32 carbon atoms in the alkyl groups, or, when at least one of R¹, R², R³, and R⁴ is different from the others, the tetraalkylammonium cation has in total at least 37 carbon atoms in the alkyl groups;
b) separating the biphasic mixture from step a) into an aqueous phase P1 and an organic phase P2;
c) optionally recycling alkene oxide which may be present in the separated organic phase P2 into reaction step a); and
d) separating 1,2-alkanediol from the aqueous phase P1 separated in step b).

2. The process of claim 1, wherein the 1,2-alkanediol is 1,2-propanediol and the corresponding alkene is propene.

3. The process of claim 1 or claim 2, wherein the tungsten polyoxometalate is a heteropolytungstate, preferably a polytungstophosphate.

4. The process of claim 3, wherein the polytungstophosphate is generated in *situ* from phosphoric acid and a water-soluble alkaline tungstate, preferably from phosphoric acid and sodium tungstate, wherein the phosphoric acid and sodium tungstate more preferably are used in a molar ratio of from 1:2 to 10:1, even more preferably in a molar ratio of from 3:1 to 8:1.

5. The process of any of the preceding claims, wherein R¹ is CH₃ and R², R³, and R⁴ each are the same, and preferably are the same C₁₂-C₁₈ alkyl, or wherein each of R¹, R², R³ and R⁴ are the same C₈-C₁₈ alkyl.

6. The process of any of the preceding claims, wherein the tetraalkylammonium cation comprises at least one of tetraoctylammonium, tetradecylammonium, tetradodecylammonium, tridodecylmethylammonium, or a mixture thereof.

7. The process of any of the preceding claims, wherein the at least one tetraalkylammonium cation is provided in form of a quaternary tetraalkylammonium salt having an anion different from the tungsten polyoxometalate, preferably in form of a tetraalkylammonium sulfate or a tetraalkylammonium methyl sulfate.

8. The process of claim 7, wherein the molar ratio of the tetraalkylammonium cation to tungsten preferably is within the range of from 1:1 to 3:1.

9. The process of any of the preceding claims, wherein the reaction in step a) is carried out in the presence of at least one solvent comprising an aromatic hydrocarbon, preferably an alkylated aromatic hydrocarbon having 7 to 12 carbon atoms.

10. The process of any of the preceding claims, wherein the pH of the aqueous phase in step a) is maintained in the range of from 1.0 to 3.5, preferably of from 2.0 to 3.0.

11. The process of any of the preceding claims, wherein the reaction mixture of step a) does not comprise any compound having an ester functionality.

12. The process of any of the preceding claims, wherein the process is a continuous process and step a) preferably is carried out in a loop reactor.

13. The process of any of the preceding claims, wherein the tungsten polyoxometalate present in the organic phase P2 separated in step b) is recycled into the reaction of step a).

14. The process of any of the preceding claims, wherein step b) is carried out in a settler vessel, wherein the biphasic mixture preferably is passed over a coalescer element comprising a structured or random packing.

15. The process of any of the preceding claims, wherein the aqueous phase P1 is separated by nanofiltration in step d) into a retentate enriched in polyoxometalate and a permeate depleted in polyoxometalate, the retentate is recycled into the reaction of step a) and 1,2-alkanediol is separated from the permeate.

## Patentansprüche

1. Ein Verfahren zur Herstellung eines 1,2-Alkandiols aus dem entsprechenden Alken und Wasserstoffperoxid umfassend die Schritte des:
a) Umsetzens des Alkens mit Wasserstoffperoxid in der Gegenwart einer katalytischen Menge von mindestens einem Wolframpolyoxometalat und mindestens einem Tetraalkylammoniumkation,
wobei die Umsetzung in einer zweiphasigen Reaktionsmischung umfassend eine wässrige Phase mit einem pH von höchstens 6 und eine organische Phase durchgeführt wird,
wobei das Tetraalkylammoniumkation die allgemeine Formel N⁺R¹R²R³R⁴ aufweist, wobei R¹, R², R³ und R⁴ Alkylgruppen sind, die jeweils unabhängig gleich oder verschieden sein können, wobei, wenn alle R¹, R², R³ und R⁴ gleich sind, das Tetraalkylammoniumkation insgesamt mindestens 32 Kohlenstoffatome in den Alkylgruppen aufweist oder, wenn mindestens eine von R¹, R², R³ und R⁴ von den anderen verschieden ist, das Tetraalkylammoniumkation insgesamt mindestens 37 Kohlenstoffatome in den Alkylgruppen aufweist;
b) Trennens der zweiphasigen Mischung aus Schritt a) in eine wässrige Phase P1 und eine organische Phase P2;
c) wahlweisen Rückführens eines Alkenoxids, welches in der abgetrennten Phase P2 vorhanden sein kann, in Reaktionsschritt a); und
d) Abtrennens des 1,2-Alkandiols aus der wässrigen Phase P1, die in Schritt b) abgetrennt wurde.

2. Das Verfahren gemäß Anspruch 1, wobei das 1,2-Alkandiol 1,2-Propandiol ist und das entsprechende Alken Propen ist.

3. Das Verfahren gemäß Anspruch 1 oder Anspruch 2, wobei das Wolframatopolyoxometalat ein Heteropolywolframat, vorzugsweise ein Polywolframatophosphat ist.

4. Das Verfahren gemäß Anspruch 3, wobei das Polywolframatophosphat in situ aus Phosphorsäure und einem wasserlöslichen Alkaliwolframat erzeugt wird, vorzugsweise aus Phosphorsäure und Natriumwolframat, wobei die Phosphorsäure und das Natriumwolframat noch bevorzugter in einem molaren Verhältnis von 1:2 bis 10:1 verwendet werden, sogar noch bevorzugter in einem molaren Verhältnis von 3:1 bis 8:1.

5. Das Verfahren gemäß irgendeinem der vorhergehenden Ansprüche, wobei R¹ CH₃ ist und R², R³ und R⁴ jeweils gleich sind und vorzugsweise das gleiche C₁₂-C₁₈-Alkyl sind oder wobei jedes von R¹, R², R³ und R⁴ das gleiche C₈-C₁₈-Alkyl ist.

6. Das Verfahren gemäß irgendeinem der vorhergehenden Ansprüche, wobei das Tetraalkylammoniumkation mindestens eines von Tetraoctylammonium, Tetradecylammonium, Tetradodecylammonium, Tridodecylmethylammonium oder eine Mischung davon umfasst.

7. Das Verfahren gemäß irgendeinem der vorhergehenden Ansprüche, wobei das mindestens eine Tetraalkylammoniumkation in Form eines quartären Tetraalkylammoniumsalzes mit einem Anion, das sich von dem Wolframpolyoxometalat unterscheidet, bereitgestellt wird, vorzugsweise in Form eines Tetraalkylammoniumsulfats oder eines Tetraalkylammoniummethylsulfats.

8. Das Verfahren gemäß Anspruch 7, wobei das molare Verhältnis des Tetraalkylammoniumkations zu Wolfram vorzugsweise im Bereich von 1:1 bis 3:1 liegt.

9. Das Verfahren gemäß irgendeinem der vorhergehenden Ansprüche, wobei die Umsetzung in Schritt a) in der Gegenwart von mindestens einem Lösemittel umfassend einen aromatischen Kohlenwasserstoff durchgeführt wird, vorzugsweise einem alkylierten aromatischen Kohlenwasserstoff mit 7 bis 12 Kohlenstoffatomen.

10. Das Verfahren gemäß irgendeinem der vorhergehenden Ansprüche, wobei der pH der wässrigen Phase in Schritt a) in einem Bereich von 1,0 bis 3,5 gehalten wird, vorzugsweise von 2,0 bis 3,0.

11. Das Verfahren gemäß irgendeinem der vorhergehenden Ansprüche, wobei die Reaktionsmischung des Schritts a) keine Verbindung mit einer Esterfunktionalität umfasst.

12. Das Verfahren gemäß irgendeinem der vorhergehenden Ansprüche, wobei das Verfahren ein kontinuierliches Verfahren ist und Schritt a) vorzugsweise in einem Schleifenreaktor durchgeführt wird.

13. Das Verfahren gemäß irgendeinem der vorhergehenden Ansprüche, wobei das Wolframpolyoxometalat, welches in der organischen Phase P2, die in Schritt b) abgetrennt wird, vorhanden ist, in die Umsetzung des Schritts a) zurückgeführt wird.

14. Das Verfahren gemäß irgendeinem der vorhergehenden Ansprüche, wobei Schritt b) in einem Absetzbehälter durchgeführt wird, wobei die zweiphasige Mischung vorzugsweise über ein Koaleszenzelement umfassend eine strukturierte oder zufällige Packung geführt wird.

15. Das Verfahren gemäß irgendeinem der vorhergehenden Ansprüche, wobei die wässrige Phase P1 durch Nanofiltration in Schritt d) in ein Retentat, angereichert an Polyoxometalat, und ein Permeat, abgereichert an Polyoxometalat, getrennt wird, das Retentat in die Umsetzung des Schritts a) zurückgeführt wird und 1,2-Alkandiol von dem Permeat abgetrennt wird.

## Revendications

1. Procédé de préparation d'un alcane-1,2-diol à partir de l'alcène correspondant et de peroxyde d'hydrogène, comportant les étapes suivantes :
a) faire réagir l'alcène avec du peroxyde d'hydrogène, en présence d'une quantité catalytique d'au moins un polyoxométalate de tungstène et d'au moins un cation tétraalkyl-ammonium,
laquelle réaction est mise en œuvre dans un mélange réactionnel biphasique comprenant une phase aqueuse dont le pH vaut au plus 6 et une phase organique,
et lequel cation tétraalkyl-ammonium a pour formule générale N⁺R¹R²R³R⁴ dans laquelle R¹, R², R³ et R⁴ représentent des groupes alkyle qui peuvent être, chacun indépendamment, identiques ou différents, étant entendu que, si les groupes représentés par R¹, R², R³ et R⁴ sont tous identiques, le cation tétraalkyl-ammonium comporte au total au moins 32 atomes de carbone dans les groupes alkyle, et que, si au moins l'un des groupes représentés par R¹, R², R³ et R⁴ est différent des autres, le cation tétraalkyl-ammonium comporte au total au moins 37 atomes de carbone dans les groupes alkyle,
b) séparer le mélange biphasique issu de l'étape (a) en une phase aqueuse P1 et une phase organique P2,
c) en option, recycler dans la réaction de l'étape (a) l'oxyde d'alcène qui peut se trouver présent dans la phase organique séparée P2,
d) et isoler l'alcane-1,2-diol à partir de la phase aqueuse P1 séparée dans l'étape (b).

2. Procédé conforme à la revendication 1, dans lequel l'alcane-1,2-diol est le propane-1,2-diol et l'alcène correspondant est le propène.

3. Procédé conforme à la revendication 1 ou 2, dans lequel le polyoxométalate de tungstène est un hétéropolytungstate, et de préférence un polytungstophosphate.

4. Procédé conforme à la revendication 3, dans lequel le polytungstophosphate est formé *in situ* à partir d'acide phosphorique et d'un tungstate de métal alcalin soluble dans l'eau, de préférence à partir d'acide phosphorique et de tungstate de sodium, étant entendu qu'il est plus avantageux d'utiliser l'acide phosphorique et le tungstate de sodium en un rapport molaire valant de 1/2 à 10/1, et mieux encore, en un rapport molaire valant de 3/1 à 8/1.

5. Procédé conforme à l'une des revendications précédentes, dans lequel R¹ représente CH₃ et R², R³ et R⁴ représentent chacun le même groupe, de préférence le même groupe alkyle en C₁₂-C₁₈, ou dans lequel R¹, R², R³ et R⁴ représentent chacun le même groupe alkyle en C₈-C₁₈,

6. Procédé conforme à l'une des revendications précédentes, dans lequel le cation tétraalkyl-ammonium comprend au moins l'un des cations tétraoctyl-ammonium, tétradécyl-ammonium, tétradodécyl-ammonium et tridodécyl-méthyl-ammonium, ou un mélange de ceux-ci.

7. Procédé conforme à l'une des revendications précédentes, dans lequel le cation tétraalkyl-ammonium, au nombre d'au moins un, est apporté sous la forme d'un sel de tétraalkyl-ammonium quaternaire dont l'anion est différent de celui du polyoxométalate de tungstène, et de préférence, sous la forme d'un sulfate de tétraalkyl-ammonium ou d'un méthyl-sulfate de tétraalkyl-ammonium.

8. Procédé conforme à la revendication 7, dans lequel le rapport molaire du cation tétraalkyl-ammonium au tungstène se trouve de préférence dans l'intervalle allant de 1/1 à 3/1.

9. Procédé conforme à l'une des revendications précédentes, dans lequel la réaction de l'étape (a) est mise en œuvre en présence d'au moins un solvant comprenant un hydrocarbure aromatique, de préférence un hydrocarbure aromatique alkylé comportant de 7 à 12 atomes de carbone.

10. Procédé conforme à l'une des revendications précédentes, dans lequel, dans l'étape (a), le pH de la phase aqueuse est maintenu dans l'intervalle allant de 1,0 à 3,5, et de préférence, de 2,0 à 3,0.

11. Procédé conforme à l'une des revendications précédentes, dans lequel le mélange réactionnel de l'étape (a) ne comprend aucun composé doté d'une fonction ester.

12. Procédé conforme à l'une des revendications précédentes, qui est un procédé continu et dans lequel l'étape (a) est de préférence mise en œuvre dans un réacteur fonctionnant en boucle.

13. Procédé conforme à l'une des revendications précédentes, dans lequel le polyoxométalate de tungstène présent dans la phase organique P2 séparée au cours de l'étape (b) est recyclé dans la réaction de l'étape (a).

14. Procédé conforme à l'une des revendications précédentes, dans lequel l'étape (b) est mise en œuvre dans une cuve à décantation, et dans lequel, de préférence, on fait passer le mélange biphasique sur un élément coalesceur comprenant un garnissage structuré ou en vrac.

15. Procédé conforme à l'une des revendications précédentes, dans lequel, dans l'étape (d), la phase aqueuse P1 est séparée, par nanofiltration, en un rétentat enrichi en polyoxométalate et un perméat appauvri en polyoxométalate, le rétentat est recyclé dans la réaction de l'étape (a) et l'alcane-1,2-diol est isolé à partir du perméat.
